# EUROPÄISCHE PATENTSCHRIFT

(11) **EP 3 003 260 B1**
(45) Veröffentlichungstag und Bekanntmachung des Hinweises auf die Patenterteilung: **01.03.2017**
(21) Anmeldenummer: 14722233.5
(22) Anmeldetag: 09.05.2014
(51) Int. Cl.: A61K 8/46, A61Q 17/04, A61K 8/895

(54) **KOSMETISCHE ZUBEREITUNG, ENTHALTEND POLYSILICONE-25 UND WASSERLÖSLICHE UV-FILTER MIT EINER SULFONSÄURE-FUNKTION**
COSMETIC PREPARATION CONTAINING POLYSILICONE-25 AND WATER-SOLUBLE UV-FILTERS WITH A SULFONIC ACID FUNCTION
PRÉPARATION COSMÉTIQUE, CONTENANT DE LA POLYSILICONE-25 ET DES FILTRES UV SOLUBLES DANS L'EAU PRÉSENTANT UNE FONCTION D'ACIDE SULFONIQUE

(30) Priorität: 28.05.2013 DE 102013209904
(43) Veröffentlichungstag der Anmeldung: 13.04.2016
(73) Patentinhaber: Beiersdorf AG, 20253 Hamburg (DE)
(72) Erfinder: MATHIEU, Claire, 22769 Hamburg (DE); KÖHLER, Manuela, 22147 Hamburg (DE)
(86) Internationale Anmeldenummer: PCT/EP2014/059523
(87) Internationale Veröffentlichungsnummer: WO 2014/191181

(56) Entgegenhaltungen:
- MOMENTIVE PERFORMANCE MATERIALS INC: "SILFORM* EOF emulsifier", MARKETING BULLETIN, März 2012 (2012-03), Seiten 1/12-12/12, XP002727858, Albany, NY, USA in der Anmeldung erwähnt
- "W/O emulsifier for suncare", IP.COM JOURNAL, IP.COM INC., WEST HENRIETTA, NY, US, 7. August 2012 (2012-08-07), XP013153055, ISSN: 1533-0001

## Beschreibung

Die vorliegende Erfindung betrifft eine kosmetische Zubereitung enthaltend Polysilicone-25 und wasserlösliche UV-Filter mit einer Sulfonsäure-Funktion.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurden daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie der Anlage 7 der deutschen Kosmetikverordnung zusammengefasst.

Die Vielzahl an kommerziell erhältlichen Sonnenschutzmitteln darf jedoch nicht darüber hinwegtäuschen, dass diese Zubereitungen des Standes der Technik eine Reihe von Nachteilen aufweisen.

Sonnenschutzmittel werden häufig in Zusammenhang mit Wassersportaktivitäten (Baden, Schwimmen, Surfen, Tauchen etc.) verwendet. Dabei tritt das Problem auf, dass das Sonnenschutzmittel bei Kontakt mit Wasser im Laufe der Zeit von der Haut abgewaschen wird. Der Lichtschutzfaktor auf der Haut geht damit zurück. Die Haut muss daher regelmäßig mit Sonnenschutzmittel nachbehandelt werden, um den UV-Schutz aufrecht zu erhalten.

Um die Anhaftung von Sonnenschutzmitteln auf der Haut zu erhöhen, werden nach dem Stand der Technik den Zubereitungen Filmbildner zugesetzt. Diese führen aber regelmäßig zu einem klebrigen, sensorisch unattraktiven Hautgefühl.

Es war daher die Aufgabe der vorliegenden Erfindung, die Nachteile des Standes der Technik zu beseitigen und ein Sonnenschutzmittel zu entwickeln, dass auch bei intensiven Kontakt mit Wasser einen hohen UV-Schutz gewährleistet. Darüber hinaus sollte dieses Sonnenschutzmittel ein attraktives, nicht klebriges Hautgefühl aufweisen.

Überraschend gelöst wird die Aufgabe durch eine kosmetische Zubereitung enthaltend
a) Polysilicone-25 und
b) wasserlösliche UV-Filter mit einer Sulfonsäure-Funktion,
dadurch gekennzeichnet, dass die Ölphase der Zubereitung Cetearylisononanoat, C12-15 Alkylbenzoat und/oder Ethylhexylcocoat enthält.

Insbesondere war es für den Fachmann überraschend und nicht vorhersehbar, dass die Zubereitungen besonders wasserfest sind und bei erfindungsgemäß besonders bevorzugten Ausführungsformen der gemessene Lichtschutzfaktor SPF nach dem Kontakt mit Wasser höher ist, als nach dem direkten Auftrag der Zubereitung auf die Haut.

Im Rahmen der vorliegenden Offenbarung ist mit Zubereitung immer die erfindungsgemäße Zubereitung gemeint, wenn nichts anderes erwähnt ist.

Zwar kennt der Stand der Technik das Marketing Bulletin "Silform EOF emulsifier EO-free W/O-Emulsifier" der Firma Momentive, doch konnte diese Produktinformation nicht den Weg zur vorliegenden Erfindung weisen wie der technical disclosure IP.com number IPCOM000220565D vom 7. August 2012.

Es ist erfindungsgemäß vorteilhaft, wenn die erfindungsgemäße Zubereitung Polysilicone-25 in einer Konzentration von 0,1 bis 2,0 Gewichts-%, bezogen auf das Gesamtgewicht der Zubereitung, enthält.

Es ist erfindungsgemäß bevorzugt, wenn die erfindungsgemäße Zubereitung Polysilicone-25 in einer Konzentration von 0,5 bis 1,0 Gewichts-%, bezogen auf das Gesamtgewicht der Zubereitung, enthält.

Das erfindungsgemäße Polysilicone-25 kann bei der Firma Momentive unter dem Handelsnamen SILFORM EOF käuflich erworben werden.

Es ist erfindungsgemäß vorteilhaft, wenn die erfindungsgemäße Zubereitung wasserlösliche UV-Filter mit einer Sulfonsäure-Funktion in einer Gesamtmenge von 0,1 bis 15 Gewichts-%, bezogen auf das Gesamtgewicht der Zusammensetzung, enthält.

Es ist erfindungsgemäß bevorzugt, wenn die erfindungsgemäße Zubereitung wasserlösliche UV-Filter mit einer Sulfonsäure-Funktion in einer Gesamtmenge von 0,5 bis 10 Gewichts-%, bezogen auf das Gesamtgewicht der Zusammensetzung, enthält.

Es ist erfindungsgemäß bevorzugt, wenn als wasserlösliche UV-Filter mit einer Sulfonsäure-Funktion 2-Phenylbenzimidazol-5-sulfonsäure und/oder deren Salze, Phenylen-1,4-bis-(2-benzimidazyl)-3,3'-5,5'-tetrasulfonsäuresalze, 1,4-di(2-oxo-10-Sulfo-3-bornylidenmethyl)-Benzol und dessen Salze, 4-(2-Oxo-3-bornylidenmethyl)benzolsulfonsäuresalze und/oder Terephthalidendicamphersulfonsäure und deren Salze eingesetzt wird.

Es ist erfindungsgemäß besonders bevorzugt, wenn als wasserlöslicher UV-Filter mit einer Sulfonsäure-Funktion 2-Phenylbenzimidazol-5-sulfonsäure und/oder deren Salze eingesetzt wird.

In einem solchen Falle ist es erfindungsgemäß vorteilhaft, wenn die erfindungsgemäße Zubereitung Phenylbenzimidazol-5-sulfonsäure und/oder deren Salze in einer Gesamtkonzentration von 0,5 bis 10 Gewichts-%, bezogen auf das Gesamtgewicht der Zubereitung, enthält.

Es ist in diesem Falle erfindungsgemäß bevorzugt, wenn die erfindungsgemäße Zubereitung Phenylbenzimidazol-5-sulfonsäure und/oder deren Salze in einer Gesamtkonzentration von 1,0 bis 5 Gewichts-%, bezogen auf das Gesamtgewicht der Zubereitung, enthält.

Als Salze werden erfindungsgemäß vorteilhaft, Natrium-, Kalium-, Trimethylammonium-, Triethylammonium- oder Triethanolamoniumsalze eingesetzt.

Es ist erfindungsgemäß vorteilhaft, wenn die erfindungsgemäße Zubereitung einen oder mehrere zusätzliche UV-Filter enthält.

Erfindungsgemäß bevorzugt handelt es sich bei der erfindungsgemäßen Zubereitung um ein kosmetisches Sonnenschutzmittel.

Es ist erfindungsgemäß vorteilhaft, wenn die erfindungsgemäße Zubereitung einen oder mehrere weitere UV-Filter gewählt aus der Gruppe der Verbindungen Phenylen-1,4-bis-(2-benzimidazyl)-3,3'-5,5'-tetrasulfonsäuresalze; 2-Phenylbenzimidazol-5-sulfonsäuresalze; 1,4-di(2-oxo-10-Sulfo-3-bornylidenmethyl)-Benzol und dessen Salze; 4-(2-Oxo-3-bornylidenmethyl)ben25 zolsulfonsäuresalze; 2-Methyl-5-(2-oxo-3-bornylidenmethyl)sulfonsäuresalze; 2,2'-Methylenbis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; 2-(4'-Diethylamino-2'-hydoxybenzoyl)-benzoesäurehexylester; Terephthalidendicamphersulfonsäure; 4-30 (Dimethylamino)-benzoesäure(2-ethylhexyl)ester; 4-(Dimethylamino)benzoesäureamylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; Homomenthylsalicylat; 2-Ethylhexyl-2-hydroxybenzoat;; Dimethicodiethylbenzalmalonat; 3-(4-(2,2-bis Ethoxycarbonylvinyl)-35 phenoxy)propenyl)-methoxysiloxan / Dimethylsiloxan - Copolymer; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoësäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin); 2,4,6-Tris-(biphenyl)-1,3,5-triazin; 2,4-Bis-(4'- Di-neopentylaminobenzalmalonat)-6-(4"-butylaminobenzoat)-5 s-triazin, 4-Dicyanomethylen-2,6-dimethyl-1,4-dihydropyridin-N-(ethyloxysulfatestersalz), Titandioxid, Zinkoxid, Merocyanine, Piperazinderivate enthält.

Erfindungsgemäß besonders bevorzugte Ausführungsformen der vorliegenden Erfindung sind dadurch gekennzeichnet, dass die Zubereitung frei ist von 3-(4-Methylbenzyliden)-campher und 2-Hydroxy-4-methoxybenzophenon (Oxybenzon).

Hinsichtlich der Einsatzkonzentrationen der UV-Filter ist es erfindungsgemäß vorteilhaft, wenn die Zubereitung UV-Filter in der Gesamtmenge von 10 bis 40 Gewichts-%, bezogen auf das Gesamtgewicht der Zubereitung, enthält. Erfindungsgemäß bevorzugt ist es, wenn die Zubereitung UV-Filter in der Gesamtmenge von 12 bis 30 Gewichts-%, bezogen auf das Gesamtgewicht der Zubereitung, enthält.

Erfindungsgemäß vorteilhafte Ausführungsformen der vorliegenden Erfindung sind auch dadurch gekennzeichnet, dass die Zubereitung einen oder mehrere Wirkstoffe gewählt aus der Gruppe der Verbindungen Gylcyrrhetinsäure, Harnstoff, Arctiin, alpha-Liponsäure, Folsäure, Phytoen, D-Biotin, Coenzym Q10, alpha-Glucosylrutin, Carnitin, Carnosin, Coffein, natürliche und/oder synthetische Isoflavonoide, Glycerylglucose, Kreatin, Kreatinin, Taurin, ß-Alanin und/oder Licochalcon A, enthält.

Erfindungsgemäß vorteilhaft liegt die erfindungsgemäße Zubereitung in Form einer W/O-Emulsion vor. Dabei ist es erfindungsgemäß bevorzugt, wenn die Zubereitung außer Polysilicone-25 keine weiteren Emulgatoren enthält.

Die Ölphase der erfindungsgemäßen Zubereitung wird vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Jojobaöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.

Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).

Ferner kann die Ölphase vorteilhaft gewählt werden aus der Gruppe der Dicaprylylether enthalten.

Es ist ferner vorteilhaft, das oder die Ölkomponenten aus der Gruppe Isoparaffine, Isoeikosan, Neopentylglykoldiheptanoat, Propylenglykoldicaprylat/dicaprat, Caprylic/Capric/Diglycerylsuccinat, Butylenglykol Dicaprylat/Dicaprat, C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid.

Vorteilhafte Ölkomponenten sind ferner z. B. Butyloctylsalicylat (beispielsweise das unter der Handelsbezeichnung *Hallbrite BHB* bei der Fa. CP Hall erhältliche), Tridecylsalicylat (welches unter der Handelsbezeichnung Cosmacol ESI bei der Fa. Sasol erhältlich ist), C12-C15 Alkylsalicylat (unter der Handelsbezeichnung Dermol NS bei der Fa. Alzo erhältlich), Hexadecylbenzoat und Butyloctylbenzoat und Gemische davon (*Hallstar AB*).

Auch beliebige Abmischungen solcher Öl- und Wachskomponenten sind vorteilhaft im Sinne der vorliegenden Erfindung einzusetzen.

Erfindungsgemäß besonders bevorzugt enthaltenden die erfindungsgemäßen Zubereitungen kein Dimethicone + Dimethicone Crosspolymer.

Die Wasserphase der erfindungsgemäßen Zubereitungen kann -neben Wasser- vorteilhaft übliche kosmetische Hilfsstoffe enthalten, wie beispielsweise Alkohole, insbesondere solche niedriger C-Zahl wie Ethanol, Isopropanol, Diole oder Polyole niedriger C-Zahl sowie deren Ether, vorzugsweise Propylenglykol, Butylenglykol, 2-Methylpropan-1,3-diol, Pentan-1,2-diol, Hexan-1,2-diol, Octan-1,2-diol, Decan-1,2-diol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, Elektrolyte, etc.. Die erfindungsgemäße Zubereitung kann vorteilhaft Konsistenzbildner (Gelbildner, Verdickungsmittel) wie beispielsweise Polyacrylate (auch quervernetzt) oder Cellulosederivate (beispielsweise Hydroxyethylcellulose) oder andere enthalten.

Es ist erfindungsgemäß bevorzugt, wenn die erfindungsgemäße Zubereitung Ethanol enthält.

Erfindungsgemäß bevorzugt ist es, wenn die erfindungsgemäße Zubereitung dadurch gekennzeichnet ist, dass die Zubereitung Propylenglycol, Butylenglycol, 2-Methylpropan-1,3-diol, 1,2-Pentandiol, 1,2-Hexandiol, 1,2-Octandiol und/oder 1,2-Decandiol enthält. Dabei werden diese Diole/Glycole erfindungsgemäß vorteilhaft in einer Gesamtmenge von 0,25 bis 4 Gewichts-%, bezogen auf das Gesamtgewicht der Zubereitung, eingesetzt.

Selbstverständlich kann die Zubereitung mit den üblichen, in der Kosmetik verwendeten Konservierungsmitteln konserviert werden.

Dabei ist es erfindungsgemäß bevorzugt, wenn die Zubereitung Phenoxyethanol enthält.

Darüber hinaus ist es erfindungsgemäß bevorzugt, wenn die Zubereitung frei ist von Parabenen.

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| **A** | **B** | **inci** |
|---|---|---|
| 0,5 | 0,5 | Polysilicone-25 |
| 39 | 39 | Aqua |
| 4,5 | 4 | Alcohol Denat. |
| 0,5 | 0,5 | Sodium Chloride |
| | | Dicaprylyl Carbonate |
| 4,84 | 4,84 | Cetearyl Isononanoate |
| 5 | 5 | C12-15 Alkyl Benzoate |
| 10 | 10 | Ethylhexyl Cocoate |
| | 0,5 | VP/Hexadecene Copolymer |
| | | Dimethicone + Dimethicone Crosspolymer |
| 0,4 | 0,4 | Parfum |
| 7,5 | 7,5 | Glycerin |
| 0,1 | 0,1 | Tocopheryl Acetate |
| 0,46 | 0,46 | Aqua + Sodium Hydroxide |
| 0,65 | 0,65 | Phenoxyethanol |
| 0,05 | 0,05 | Piroctone Olamine |
| 1 | 1 | Aqua + Trisodium EDTA |
| 3,5 | 3,5 | Homosalate |
| 9 | 9 | Octocrylene |
| 1,5 | 1,5 | Ethylhexyl Salicylate |
| 4,5 | 4,5 | Butyl Methoxydibenzoylmethane |
| 2 | 2 | Titanium Dioxide (nano) + Trimethoxycaprylylsilane |
| 3,5 | 3,5 | Bis-Ethylhexyloxyphenol Methoxyphenyl Triazine |
| 1,5 | 1,5 | Phenylbenzimidazole Sulfonic Acid |
| **93% (79-106%)** | **99% (81-118%)** | Wasserfestiqkeit |
| 83-114 | 80-128 | SPF vor dem Baden |
| 73-112 | 86-122 | SPF nach dem Baden |

## Patentansprüche

1. Kosmetische Zubereitung enthaltend
a) Polysilicone-25 und
b) wasserlösliche UV-Filter mit einer Sulfonsäure-Funktion,
**dadurch gekennzeichnet, dass** die Ölphase der Zubereitung Cetearylisononanoat, C12-15 Alkylbenzoat und/oder Ethylhexylcocoat enthält.

2. Kosmetische Zubereitung nach Anspruch 1, **dadurch gekennzeichnet, dass** die Zubereitung Polysilicone-25 in einer Konzentration von 0,1 bis 2,0 Gewichts-%, bezogen auf das Gesamtgewicht der Zubereitung, enthält.

3. Kosmetische Zubereitung nach einem der vorhergehenden Ansprüche, **dadurch gekennzeichnet, dass** als wasserlösliche UV-Filter mit einer Sulfonsäure-Funktion 2-Phenylbenzimidazol-5-sulfonsäure und/oder deren Salze, Phenylen-1,4-bis-(2-benzimidazyl)-3,3'-5,5'-tetrasulfonsäuresalze, 1,4-di(2-oxo-10-Sulfo-3-bornylidenmethyl)-Benzol und dessen Salze, 4-(2-Oxo-3-bornylidenmethyl)benzolsulfonsäuresalze und/oder Terephthalidendicamphersulfonsäure und deren Salze eingesetzt wird.

4. Kosmetische Zubereitung nach einem der vorhergehenden Ansprüche, **dadurch gekennzeichnet, dass** die Zubereitung wasserlösliche UV-Filter mit einer Sulfonsäure-Funktion in einer Gesamtmenge von 0,5 bis 10 Gewichts-%, bezogen auf das Gesamtgewicht der Zusammensetzung, enthält.

5. Kosmetische Zubereitung nach einem der vorhergehenden Ansprüche, **dadurch gekennzeichnet, dass** als wasserlösliche UV-Filter mit einer Sulfonsäure-Funktion 2-Phenylbenzimidazol-5-sulfonsäure und/oder deren Salze eingesetzt wird.

6. Kosmetische Zubereitung nach Anspruch 5, **dadurch gekennzeichnet, dass** die Zubereitung Phenylbenzimidazol-5-sulfonsäure und/oder deren Salze in einer Gesamtkonzentration von 0,5 bis 10 Gewichts-%, bezogen auf das Gesamtgewicht der Zubereitung, enthält.

7. Kosmetische Zubereitung nach einem der vorhergehenden Ansprüche, **dadurch gekennzeichnet, dass** die Zubereitung einen oder mehrere UV-Filter enthält, gewählt aus der Gruppe der Verbindungen 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; 4-(tert.-Butyl)-4'-methoxydibenzoylmethan; 2-Ethylhexyl-2-cyano-3,3-diphenylacrylat; 4-(Dimethylamino)-benzoesäure(2-ethylhexyl)ester; 4-(Dimethylamino)benzoesäure-amylester; 4-Methoxybenzalmalonsäuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester; Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan / Dimethylsiloxan - Copolymer; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoësäure-tris-(2-ethylhexylester) (auch: 2,4,6-Tris-[anilino-(p-carbo-2'-ethyl-1'-hexyloxy)]-1,3,5-triazin (INCI: Ethylhexyl Triazone); 2,4-Bis-{[4-(2-ethyl-hexyloxy)-2-hydroxy]-phenyl}-6-(4-methoxyphenyl)-1,3,5-triazin; 2,4,6-Tribiphenyl-4-yl-1,3,5-triazin; Merocyanine.

8. Kosmetische Zubereitung nach einem der vorhergehenden Ansprüche, **dadurch gekennzeichnet, dass** die Zubereitung einen oder mehrere Wirkstoffe gewählt aus der Gruppe der Verbindungen Gylcyrrhetinsäure, Harnstoff, Arctiin, alpha-Liponsäure, Folsäure, Phytoen, D-Biotin, Coenzym Q10, alpha-Glucosylrutin, Carnitin, Carnosin, Coffein, natürliche und/oder synthetische Isoflavonoide, Glycerylglucose, Kreatin, Kreatinin, Taurin, ß-Alanin und/oder Licochalcon A, enthält.

9. Kosmetische Zubereitung nach einem der vorhergehenden Ansprüche, **dadurch gekennzeichnet, dass** die Zubereitung Propylenglycol, Butylenglycol, 2-Methylpropan-1,3-diol, 1,2-Pentandiol, 1,2-Hexandiol, 1,2-Octandiol und/oder 1,2-Decandiol enthält.

10. Kosmetische Zubereitung nach einem der vorhergehenden Ansprüche, **dadurch gekennzeichnet, dass** die Zubereitung in Form einer W/O-Emulsion vorliegt.

11. Kosmetische Zubereitung nach einem der vorhergehenden Ansprüche, **dadurch gekennzeichnet, dass** die Zubereitung Ethanol enthält.

12. Kosmetische Zubereitung nach einem der vorhergehenden Ansprüche, **dadurch gekennzeichnet, dass** die Zubereitung frei ist von Parabenen.

## Claims

1. Cosmetic preparation containing
a) polysilicone-25 and
b) water-soluble UV filters with a sulphonic acid function,
**characterized in that** the oil phase of the preparation contains cetearyl isononanoate, C12-15 alkyl benzoate and/or ethylhexyl cocoate.

2. Cosmetic preparation according to Claim 1, **characterized in that** the preparation contains polysilicone-25 at a concentration of 0.1 to 2.0% by weight, based on the total weight of the preparation.

3. Cosmetic preparation according to either of the preceding claims, **characterized in that** the water-soluble UV filter with a sulphonic acid function used is 2-phenylbenzimidazole-5-sulphonic acid and/or salts thereof, phenylene-1,4-bist(2-benzimidazyl)-3,3',5,5'-tetrasulphonic acid salts, 1,4-di(2-oxo-10-sulpho-3-bornylidenemethyl)benzene and salts thereof, 4-(2-oxo-3-bornylidenemethyl)benzenesulphonic acid salts and/or terephthalidenedicamphorsulphonic acid and salts thereof.

4. Cosmetic preparation according to any of the preceding claims, **characterized in that** the preparation contains water-soluble UV filters with a sulphonic acid function in a total amount of 0.5 to 10% by weight, based on the total weight of the composition.

5. Cosmetic preparation according to any of the preceding claims, **characterized in that** the water-soluble UV filter with a sulphonic acid function used is 2-phenylbenzimidazole-5-sulphonic acid and/or salts thereof.

6. Cosmetic preparation according to Claim 5, **characterized in that** the preparation contains phenylbenzimidazole-5-sulphonic acid and/or salts thereof at a total concentration of 0.5 to 10% by weight, based on the total weight of the preparation.

7. Cosmetic preparation according to any of the preceding claims, **characterized in that** the preparation contains one or more UV filters, selected from the group of compounds 2-methyl-5-(2-oxo-3-bornylidenemethyl)sulphonic acid salts; 2,2'-methylenebis(6-(2H-benzotriazol-2-yl)-4-(1,1,3,3-tetramethylbutyl)phenol); 2-(2H-benzotriazol-2-yl)-4-methyl-6-[2-methyl-3-[1,3,3,3-tetramethyl-1-[(trimethylsilyl)oxy]disiloxanyl]propyl]phenol; 3-(4-methylbenzylidene)camphor; 3-benzylidenecamphor; ethylhexyl salicylate; 4-(tert-butyl)-4'-methoxydibenzoylmethane; 2-ethylhexyl 2-cyano-3,3-diphenylacrylate; 2-ethylhexyl 4-(dimethylamino)benzoate; amyl 4-(dimethylamino)benzoate; di(2-ethylhexyl) 4-methoxybenzalmalonate; 2-ethylhexyl 4-methoxycinammate; isoamyl 4-methoxycinnamate; 2-hydroxy-4-methoxybenzophenone, 2-hydroxy-4-methoxy-4'-methylbenzophenone; 2,2'-dihydroxy-4-methoxybenzophenone; hexyl 2-(4'-diethylamino-2'-hydroxybenzoyl)benzoate; homomenthyl salicylate; 2-ethylhexyl 2-hydroxybenzoate: dimethicodiethylbenzalmalonate; 3-(4-(2,2-bisethoxycarbonylvinyl)phenoxy)propenyl)methoxysiloxane / dimethylsiloxane copolymer; dioctylbutylamidotriazone (INCI: Diethylhexyl Butamido Triazone); 2,4-bis[5-1(dimethylpropyl)benzoxazol-2-yl-(4-phenyl)lmino]-6-(2-ethylhexyl)imino-1,3,5-triazine with (CAS No. 288254-16-0); tris(2-ethylhexyl) 4,4',4"-(1,3,5-triazine-2,4,6-triyltriimino)trisbenzoate (also: 2,4,6-tris[anilino(p-carbo-2'-ethyl-1'-hexyloxy)]-1,3,5-triazine (INCI: Ethylhexyl Triazone); 2,4-bis{[4-(2-ethylhexyloxy)-2-hydroxy]phenyl}-6-(4-methoxyphenyl)-1,3,5-triazine; 2,4,6-tribiphenyl-4-yl-1,3,5-triazine; merocyanine.

8. Cosmetic preparation according to any of the preceding claims, **characterized in that** the preparation contains one or more active ingredients selected from the group of the compounds glycyrrhetic acid, urea, arctiin, alpha-lipoic acid, folic acid, phytoene, D-biotin, coenzyme Q10, alpha-glucosylrutin, carnitine, carnosine, caffeine, natural and/or synthetic isoflavonoids, glycerylglucose, creatine, creatinine, taurine, β-alanine and/or licochalcone A.

9. Cosmetic preparation according to any of the preceding claims, **characterized in that** the preparation contains propylene glycol, butylene glycol, 2-methylpropane-1,3-diol, 1,2-pentanediol, 1,2-hexanediol, 1,2-octanediol and/or 1,2-decanediol.

10. Cosmetic preparation according to any of the preceding claims, **characterized in that** the preparation is in the form of a W/O emulsion.

11. Cosmetic preparation according to any of the preceding claims, **characterized in that** the preparation contains ethanol.

12. Cosmetic preparation according to any of the preceding claims, **characterized in that** the preparation is free of parabens.

## Revendications

1. Préparation cosmétique contenant :
a) de la polysilicone 25 et
b) des filtres UV solubles dans l'eau à fonction acide sulfonique,
**caractérisée en ce que** la phase huileuse de la préparation contient de l'isononanoate de cétéaryle, du benzoate d'alkyle en C12-15 et/ou du cocoate d'éthylhexyle.

2. Préparation cosmétique selon la revendication 1, **caractérisée en ce que** la préparation contient de la polysilicone 25 en une concentration de 0,1 à 2,0 % en poids, par rapport au poids total de la préparation.

3. Préparation cosmétique selon l'une quelconque des revendications précédentes, **caractérisée en ce que** l'acide 2-phénylbenzimidazole-5-sulfonique et/ou ses sels, les sels de l'acide phénylène-1,4-bis-(2-benzimidazyl)-3,3'-5,5'-tétrasulfonique, le 1,4-di(2-oxo-10-sulfo-3-bornylidène-méthyl)-benzène et ses sels, les sels de l'acide 4-(2-oxo-3-bornylidène-méthyl)benzène-sulfonique et/ou l'acide téréphtalidène-dicamphre-sulfonique et ses sels sont utilisés en tant que filtres UV solubles dans l'eau à fonction acide sulfonique.

4. Préparation cosmétique selon l'une quelconque des revendications précédentes, **caractérisée en ce que** la préparation contient des filtres UV solubles dans l'eau à fonction acide sulfonique en une quantité totale de 0,5 à 10 % en poids, par rapport au poids total de la composition.

5. Préparation cosmétique selon l'une quelconque des revendications précédentes, **caractérisée en ce que** l'acide 2-phénylbenzimidazole-5-sulfonique et/ou ses sels sont utilisés en tant que filtres UV solubles dans l'eau à fonction acide sulfonique.

6. Préparation cosmétique selon la revendication 5, **caractérisée en ce que** la préparation contient de l'acide phénylbenzimidazole-5-sulfonique et/ou ses sels en une concentration totale de 0,5 à 10 % en poids, par rapport au poids total de la préparation.

7. Préparation cosmétique selon l'une quelconque des revendications précédentes, **caractérisée en ce que** la préparation contient un ou plusieurs filtres UV, choisis dans le groupe des composés sels de l'acide 2-méthyl-5-(2-oxo-3-bornylidène-méthyl)sulfonique; 2,2'-méthylène-bis-(6-(2H-benzotriazol-2-yl)-4-(1,1,3,3-tétraméthylbutyl)-phénol) ; 2-(2H-benzotriazol-2-yl)-4-méthyl-6-[2-méthyl-3-[1,3,3,3-tétraméthyl-1-[(tri-méthylsilyl)oxy]disiloxanyl]propyl]-phénol ; 3-(4-méthylbenzylidène)camphre ; 3-benzylidène-camphre ; salicylate d'éthylhexyle ; 4-(tert.-butyl)-4'-méthoxydibenzoylméthane ; acrylate de 2-éthylhexyl-2-cyano-3,3-diphényle ; ester (2-éthylhexylique) de l'acide 4-(diméthylamino)-benzoïque ; ester amylique de l'acide 4-(diméthylamino)benzoïque ; ester (2-éthylhexylique) de l'acide 4-méthoxybenzalmalonique ; ester (2-éthylhexylique) de l'acide 4-méthoxycinnamique ; ester isoamylique de l'acide 4-méthoxycinnamique ; 2-hydroxy-4-méthoxybenzophénone, 2-hydroxy-4-méthoxy-4'-méthylbenzophénone ; 2,2'-dihydroxy-4-méthoxybenzophénone ; ester hexylique de l'acide 2-(4'-diéthylamino-2'-hydoxybenzoyl)-benzoïque ; salicylate d'homomenthyle ; 2-hydroxybenzoate de 2-éthylhexyle ; benzalmalonate de diméthicodiéthyle ; copolymère de 3-(4-(2,2-bis-éthoxycarbonylvinyl)-phénoxy)propényl)-méthoxysiloxane/diméthylsiloxane ; dioctylbutylamidotriazone (INCI : diéthylhexyl-butamidotriazone) ; 2,4-bis-[5-1(diméthyl-propyl)benzoxazol-2-yl-(4-phényl)-imino]-6-(2-éthylhexyl)-imino-1,3,5-triazine de n° CAS 288254-16-0 ; ester tris-(2-éthylhexylique) de l'acide 4,4',4"-(1,3,5-triazin-2,4,6-triyltriimino)-tris-benzoïque (aussi : 2,4,6-tris-[anilino-(p-carbo-2'-éthyl-1'-hexyloxy)]-1,3,5-triazine (INCI : éthylhexyltriazone) ; 2,4-bis-{[4-(2-éthyl-hexyloxy)-2-hydroxy]-phényl}-6-(4-méthoxyphényl)-1,3,5-triazine; 2,4,6-tribiphényl-4-yl-1,3,5-triazine ; mérocyanine.

8. Préparation cosmétique selon l'une quelconque des revendications précédentes, **caractérisée en ce que** la préparation contient un ou plusieurs agents actifs choisis dans le groupe des composés acide glycyrrhizique, urée, arctiine, acide alpha-lipoïque, acide folique, phytoène, D-biotine, coenzyme Q10, alpha-glucosylrutine, carnitine, carnosine, cafféine, isoflavonoïdes naturels et/ou synthétiques, glycérylglucose, créatine, créatinine, taurine, β-alanine et/ou licochalcone A.

9. Préparation cosmétique selon l'une quelconque des revendications précédentes, caractériséee en ce que la préparation contient du propylène glycol, du butylène glycol, du 2-méthylpropane-1,3-diol, du 1,2-pentanediol, du 1,2-hexanediol, du 1,2-octanediol et/ou du 1,2-décanediol.

10. Préparation cosmétique selon l'une quelconque des revendications précédentes, **caractérisée en ce que** la préparation se présente sous la forme d'une émulsion E/H.

11. Préparation cosmétique selon l'une quelconque des revendications précédentes, **caractérisée en ce que** la préparation contient de l'éthanol.

12. Préparation cosmétique selon l'une quelconque des revendications précédentes, **caractérisée en ce que** la préparation est exempte de parabènes.
